# EUROPEAN PATENT SPECIFICATION

(11) **EP 1 283 064 B1**
(45) Date of publication and mention of the grant of the patent: **23.05.2007**
(21) Application number: 02016979.3
(22) Date of filing: 02.08.2002
(51) Int. Cl.: A61M 1/34, A61M 1/36

(54) **Blood processing system**
Blutbehandlungssystem
Appareil de traitement du sang

(30) Priority: 10.08.2001 JP 2001243208
(43) Date of publication of application: 12.02.2003
(73) Proprietor: KURARAY CO., LTD., Kurashiki-shi, Okayama 710-8622 (JP)
(72) Inventor: Inoue, Masao, Kurashiki-shi, Okayama 710-8622 (JP); Ike, Akihiro, Chuo-ku, Tokyo 103-8254 (JP); Nakaji, Shuhei, Kurashiki-shi, Okayama 710-8622 (JP)
(74) Representative: Vossius & Partner

(56) References cited:
- EP-A- 0 834 329
- US-A- 4 647 378
- US-A- 5 460 715
- US-A- 6 136 593
- PATENT ABSTRACTS OF JAPAN vol. 014, no. 456 (C-0765), 2 October 1990 (1990-10-02) & JP 02 182265 A (KANEGAFUCHI CHEM IND CO LTD), 16 July 1990 (1990-07-16)

## Description

The present invention relates to a blood processing system including an extracorporeal circulating circuit having a plasma purifying unit for performing purification of a plasma that has been separated from patient's blood by a plasma separating unit and for returning the purified plasma to the patient with separated blood cell.

The system has long been well known in which for purification of the blood, a plasma is separated from the blood by the use of a centrifugal separating unit and is subsequently purified by a plasma purifying unit before it is returned to the centrifugal separating unit. In this extracorporeal circulating system, a fluid circuit extending from the centrifugal separating unit to the plasma purifying unit and a fluid return circuit through which the purified plasma is returned to the centrifugal separating unit are provided with a plasma storage bag and a purified plasma reservoir, respectively. A plasma feed pump is disposed downstream of the plasma storage bag and a purified plasma feed pump is also employed for feeding the purified plasma from the plasma purifying unit. A control unit is operatively associated with the plasma feed pump and the purified plasma feed pump so that the surface level of the plasma within the plasma storage bag as detected by a liquid level detector provided in the plasma storage bag can be kept within a predetermined range. See, for example, the JP-A-60-256465 and JP-A-1-104272.

US 6 136 593 relates to an astrocyte apparatus for bio-processing a circulating fluid and particularly to a container having astrocytes for use in removing toxins from a biological fluid. The container includes a first port for receiving the biological fluid and a second port through which the biological fluid exits the container. Moreover, a method for treating a biological fluid by contacting it with astrocytes is discussed. The apparatus and method according to US 6 136 593 make use of a reservoir bag.

However, with the known blood processing system, the following problems have been found and are, hence, strongly desired to be resolved.
1)So long as the liquid surface level is detected within the plasma storage bag, no stable correlation between the liquid surface level and the actual amount of the plasma contained in the plasma storage bag can be secured because of deformation in shape of the plasma storage bag such as swelling and/or flattening. If as a result of change in shape of the plasma storage bag the surface level is erroneously detected, the plasma feed pump will be controlled irrespective of the actual amount of the plasma remaining within the plasma storage bag, resulting in unnecessarily excessive plasma processing. In such case, it is necessary to manually rectify the deformed shape of the plasma storage bag and/or the operating speed of the plasma feed pump and this is indeed cumbersome and time-consuming.
2) Even when as a result of occurrence of an abnormality during the blood purifying process the plasma feed pump is brought to a halt or is operated at a low flow rate, flow of the plasma from the centrifugal separating unit continues and, accordingly the amount of the plasma stored in the plasma storage bag increases and the plasma storage bag may rupture in the event of the worst case.
3) Considering that the plasma storage bag is generally used with no priming performed, there is a high possibility that residues brought about as a result of sterilization of the plasma storage bag may be dissolved into the plasma being processed.

In view of the foregoing, the present invention has been devised to substantially eliminate the foregoing problems inherent in the conventional apparatus in which the plasma separated from the blood by the centrifugal separating unit is substantially purified and has for its object to provide a highly reliable and safe, high functional blood processing system that is effective to accomplish:
a) an improvement in operability achieved by the fact that an automatic control is performed to substantially equalize the flow rate of the separated plasma from the plasma separating unit to the flow rate of the plasma fed by the plasma feed pump;
b) an improvement in safety factor associated with the plasma purifying process in the event of an occurrence of abnormality; and
c) performance of the plasma purifying process with no plasma storage bag employed.
The object underlying the present invention is achieved by the features of the claims.

The inventors of the present invention have conducted a series of studies to achieve the foregoing objects and have found that those objects can be attained by designing the blood processing system in the following manner. Specifically, in the system for purifying the plasma separated from the blood by the plasma separating unit, the flow rate achieved by the plasma feed pump is automatically controlled to render the plasma inlet pressure, measured by a pressure gauge disposed in a plasma introducing fluid circuit, to fall within a predetermined range with respect to a preset pressure, so that the amount of the plasma to be supplied and the flow rate achieved by the plasma feed pump can be continuously controlled to coordinate with each other. Also, when as a result of occurrence of an abnormality during the blood purifying process the plasma feed pump is brought to a halt, the use is made of a fluid circuit for supplying the plasma from the plasma purifying unit back to the plasma separating unit without the plasma being processed, so that any possible closure of the extracorporeal circulating circuit can be avoided. Moreover, the use of the plasma storage bag which is considered an excessive use is eliminated because measurement of the surface level in the plasma storage bag hitherto done in the conventional system is superseded by pressure measurement in the extracorporeal circulation circuit.

More specifically, the present invention provides a blood processing system for purifying a plasma in a blood which includes a plasma separating unit for separating a plasma from a blood; a plasma purifying unit for purifying the separated plasma; a plasma introducing fluid circuit for introducing the separated plasma into the plasma purifying unit; a plasma feed pump disposed in the plasma introducing fluid circuit and having a plasma intake port and a plasma discharge port; a plasma inlet pressure gauge for measuring a pressure of the plasma at the plasma intake port of the plasma feed pump; a plasma return fluid circuit for returning the plasma, which has been purified by the plasma purifying unit, back to the plasma separating unit; a bypass fluid circuit extending between the plasma introducing fluid circuit and the plasma return fluid circuit for bypassing the plasma feed pump and the plasma purifying unit; a valve disposed in the bypass circuit for opening the bypass circuit in the event of an occurrence of operative abnormality in the blood processing system; and a control unit for controlling the plasma feed pump in reference to the plasma inlet pressure measured by the plasma inlet pressure gauge to render the plasma inlet pressure to fall within a predetermined range with respect to a preset pressure.

Thus, the present invention provides a highly reliable and safe, high functional blood processing system that is effective to accomplish an improvement in operability achieved by the fact that an automatic control is performed to substantially equalize the flow rate of the separated plasma from the plasma separating unit to the flow rate of the plasma fed by the plasma feed pump; an improvement in safety factor associated with the plasma purifying process in the event of an occurrence of abnormality; and performance of the plasma purifying process with no plasma storage bag employed.

In a preferred embodiment, the control unit has a function of controlling the plasma feed pump to render the plasma inlet pressure to fall within the range of, for example, ±5 mmHg with respect to the preset pressure.

In a preferred embodiment, the control unit has a function of controlling the plasma feed pump to render the flow of the plasma discharged from the plasma feed pump to be within a range of, for example, ±5 ml/min. with respect to the flow of the plasma from the plasma separating unit.

The blood processing system of the present invention may further include a pressure gauge disposed between the plasma feed pump and the plasma purifying unit for detecting a plasma purifier pressure, in which case in the event that the plasma purifier pressure exceeds a predetermined value, the control unit halts the plasma feed pump and causes the valve on the bypass fluid circuit to open.

In one preferred embodiment, the blood processing system of the present invention may also include a pressure gauge disposed in the plasma return fluid circuit for detecting a pressure of the plasma being returned, in which case in the event that the pressure of the plasma being returned exceeds a predetermined value the control unit halts the plasma feed pump and causes the valve on the bypass fluid circuit to open.

In a preferred embodiment of the present invention, in the event that the plasma feed pump is halted, the control unit may have a function of causing the valve on the bypass fluid circuit to open.

In another preferred embodiment, the plasma purifying unit may be selected from the group consisting of a plasma component fractionating membrane module and a plasma component adsorbent unit.

In a further preferred embodiment, the plasma purifying unit may include the plasma component fractionating membrane module. In such case, the blood processing system may furthermore include a filtrate feed pump operable in association with the plasma feed pump for draining a filtrate separated from the plasma by the plasma component fractionating membrane module.

In a still further preferred embodiment, where the plasma purifying unit may include the plasma component fractionating membrane module, the blood processing system of the present invention may also include a supplementary liquid supply circuit for supplying a supplementary liquid to the plasma component fractionating membrane module by means of the filtrate feed pump to thereby supplement a filtrated plasma with the supplementary liquid in a quantity corresponding to the quantity of the filtrate separated from the plasma.

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:
Fig. 1 illustrates a diagram showing a fluid flow circuit of a plasma component adsorbing apparatus according to a preferred embodiment of the present invention; and
Fig. 2 illustrates a diagram showing the fluid flow circuit of the plasma component adsorbing apparatus according to another preferred embodiment of the present invention.

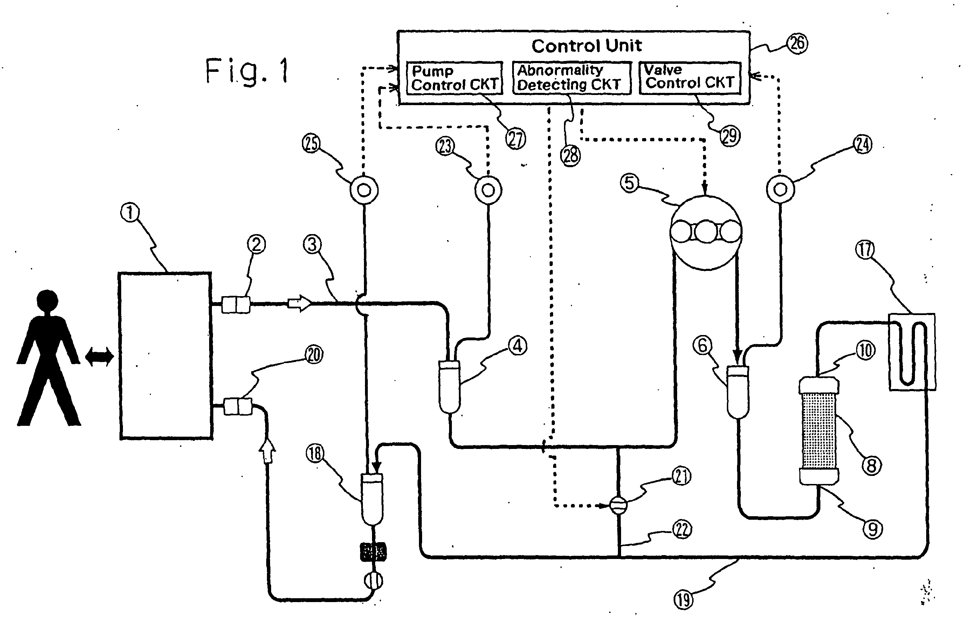

Referring first to Fig. 1 showing a blood processing system according to a preferred embodiment of the present invention. The illustrated blood processing system is a plasma component adsorbing system and includes a plasma separating unit 1 for separating blood, drawn from a patient to be treated, into a blood cell and a plasma and the plasma is supplied into a plasma introducing fluid circuit 3 through a plasma inlet 2. The plasma separating unit 1 may be, for example, a centrifugal separator such as "COBE Spectra", "Baxter CS-3000 Plus", or "HAEMONETICS MCS", or a membrane separator such as "KM-8100N" available from Kuraray Co., Ltd.

The plasma introducing circuit 3 extends from the plasma inlet 2 to a plasma intake port 9 of a plasma component adsorbent unit 8, which is a sort of a blood purifying unit, through a plasma inlet drip chamber 4, then through a plasma feed pump 5 and finally through a plasma purifier drip chamber 6. The plasma adsorbing system also include a plasma return fluid circuit 19 extending from a plasma outlet port 10 of the plasma component adsorbent unit 8 to a plasma return outlet 20 through a heater 17 and then through a plasma return drip chamber 18. A bypass fluid circuit 22 for bypassing the plasma feed pump 5 and the plasma component adsorbent unit 8 is disposed between a portion of the plasma introducing fluid circuit 3, which extends between the plasma inlet drip chamber 4 and the plasma feed pump 5, and a portion of the plasma return fluid circuit 19 which extends between the heater 17 and the plasma return drip chamber 18. This bypass fluid circuit 22 has a circuit switching valve 21 disposed thereon. In this way, an extracorporeal circulating circuit is thus completed.

Also, for detecting the pressure at various portions of the extracorporeal circulating circuit, the plasma adsorbing system also includes a plasma inlet pressure gauge 23 fluid-connected with the plasma inlet drip chamber 4 for measuring the pressure at an intake port of the feed pump 5, a plasma purifier pressure gauge 24 fluid-connected with the plasma purifier drip chamber 6 for detecting the pressure at a discharge port of the feed pump 5, and a plasma return pressure gauge 25 fluid connected with the plasma return drip chamber 18 for measuring the pressure in the plasma return fluid circuit 19.

The plasma component adsorbent unit 8 referred to above is of a type having a mass of adsorbent material filled in a column and is chosen to suit to a particular type of materies morbi that is desired to be removed. While any of various adsorbing methods such as, for example, a physical adsorption, a chemical adsorption and an affinity adsorption are available depending on the characteristics of the adsorbent material, selection of one of those various adsorbing methods employed in the plasma component adsorbent unit 8 is preferably made with a view to the use of a particular adsorbent material capable of exhibiting an excellent adsorbing performance with respect to target materials desired to be removed and being non-specific to useful material. The adsorbent material to be used in the plasma component adsorbent unit 8 may be made up of a mass of beads or fibers.

As the separated plasma is supplied from the plasma separating unit 1, the plasma inlet pressure increases. This plasma inlet pressure is measured by the plasma inlet pressure gauge 23 at all times and monitored by a control unit 26 including a pump control circuit 27 built therein for automatically controlling the flow of the plasma feed pump 5 so that the measured pressure can fall within a predetermined range with respect to a preset pressure.

By measuring the plasma inlet pressure accurately and controlling the flow of the plasma feed pump 5 so that the plasma inlet pressure can fall within the predetermined range with respect to the preset pressure, it is possible to process the plasma at a flow rate consistent with change in the amount of the separated plasma supplied from the plasma separating unit 1. In the practice of the present invention, it is preferred that the measured pressure be controlled to be within the range of ±5 mmHg and more preferably ±2 mmHg with respect to the preset pressure. At the same time the flow of the plasma discharged from the plasma feed pump 5 is preferably within the range of ±5 ml/min, and more preferably ±3 ml/min, with respect to the flow of the plasma from the plasma separating unit 1.

The flow of the plasma discharged from the plasma feed pump 5 is to be determined by the control unit 26 in reference to the number of revolutions of the plasma feed pump 5 and the pressure measured by the plasma purifier pressure gauge 24, whereas the flow of the plasma emerging from the plasma separating unit 1 is determined by the control unit 26 in reference to the number of revolutions of the plasma feed pump 5 and the pressure measured by the plasma inlet pressure gauge 23.

Even the plasma purifier pressure and the plasma return pressure are monitored by the control unit 26 through the plasma purifier pressure gauge 24 and the plasma return pressure gauge 25, respectively, so that in the event that each of those pressures exceeds a respective predetermined value, an abnormality detecting circuit 28 built in the control unit 26 detects such event with the pump control circuit 27 in the control unit 26 consequently causing the plasma feed pump 5 to halt. At the same time, a valve control circuit 29 built in the control unit 26 causes the valve 21 in the bypass circuit to open. Accordingly, with no need to interrupt the flow of the separated plasma from the plasma separating unit 1, the system can deal with such an abnormality. Also, in the event that the plasma feed pump 5 is halted by reason of any other system malfunction and/or an erroneous manual operation, the valve 21 in the bypass circuit 22 can be opened by the control unit 26 and, accordingly, it is possible to avoid the extracorporeal circulating circuit from being closed. In other words, the bypass circuit 22 is opened when the system is brought in an alert condition as a result of occurrence of an abnormality or malfunction.

In view of the foregoing, the blood processing system of the present invention is preferably equipped with any suitable warning device capable of providing an audio and/or video warning indication, such as a blinking lamp and/or an alarm, to the attendant operator in the event that the valve 21 in the bypass circuit 22 is opened.

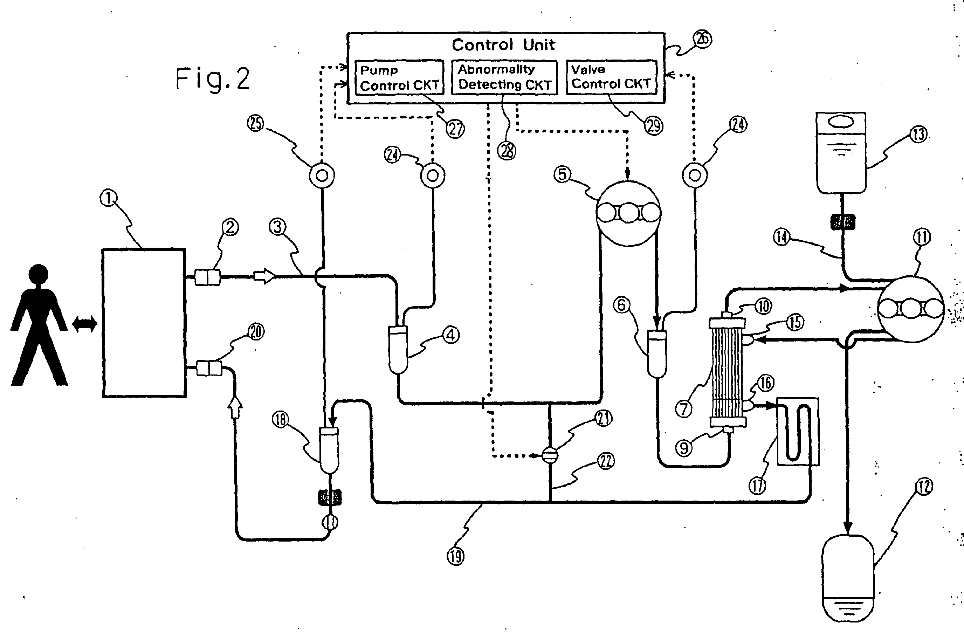

Referring now to Fig. 2 showing a blood processing system according to another preferred embodiment of the present invention, the illustrated blood processing system is a plasma purifying and exchange transfusing system. The plasma purifying unit employed in the system shown in Fig. 2 is a membrane module 7, and the plasma introducing fluid circuit 3 extending from the plasma separating unit 1 is fluid-connected with a plasma intake port 9 of this membrane module 7 in a manner similar to that with the adsorbent unit 8 shown in Fig. 1. The membrane module 7 is operable to filter the plasma and has a filtrate discharge port 10 from which a liquid component separated from the plasma is discharged and a plasma outlet port 16 from which the plasma having been so purified emerges into the plasma return fluid circuit 19 through the heater 17. A filtrate discharge circuit fluid-connected with the filtrate discharge port 10 of the membrane module 7 is fluid-connected with a filtrate disposal container 12 through a filtrate feed pump 11.

The filtrate feed pump 11 is also fluid connected with an upstream portion of a supplementary liquid supply circuit 14 that extends from a supplementary liquid reservoir 13, while a downstream portion of the supplementary liquid supply circuit 14 that extends from the filtrate feed pump 11 is fluid-connected with a supplementary liquid feed port 15 of the membrane module 7. The supplementary liquid feed circuit 14 is used so that a supplementary liquid from the supplementary liquid reservoir 13 can be supplied into the membrane module 7 and be subsequently mixed with the plasma in a quantity corresponding to the quantity of the filtrate separated from the plasma. This supplementary liquid is generally employed in the form of a physiologically compatible fluid substitute.

The plasma return fluid circuit 19 extends from the plasma outlet port 16 of the membrane module 7, from which the purified plasma emerges, to the plasma return port 20 in a manner similar to that shown in Fig. 1.

Similar to the previously described embodiment, the bypass fluid circuit 22 for bypassing the plasma feed pump 5 and the plasma purifying and exchange transfusing unit 7 is disposed between the portion of the plasma introducing fluid circuit 13, which extends between the plasma inlet drip chamber 4 and the plasma feed pump 5, and the portion of the plasma return fluid circuit 19 which extends between the heater 17 and the plasma return drip chamber 18. This bypass fluid circuit 22 has the circuit switching valve 21 disposed thereon, which when closed establishes a bypass circuit. Also, the plasma adsorbing system also includes the plasma inlet pressure gauge 23 fluid-connected with the plasma inlet drip chamber 4, the plasma purifier pressure gauge 24 fluid-connected with the plasma purifier drip chamber 6, and the plasma return pressure gauge 25 fluid-connected with the plasma return drip chamber 18.

The membrane module 7 referred to above is of a type having a plasma component fractionating membrane built therein, which membrane is preferably in the form of a hollow fiber or a plain membrane. The plasma component fractionating membrane is operable to separate the plasma component selectively into a high molecular component and a low molecular component, and the molecular weight to be fractionated can be set to any desired value depending on the molecular weight of the target materies morbi that is desired to be fractionated. Also, for the plasma component fractionating membrane, a homogeneous micropored membrane, a microfiltration membrane or a so-called asymmetric structural membrane made up of a porous support layer and a micropored structural layer can be generally employed. Material for this membrane may include a polyvinyl alcohol (PVA) polymer, ethylene vinyl alcohol (EVA) polymer, a cellulose derivative such as, for example, cellulose diacetate, or polypropylene.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention. Accordingly, such changes and modifications are, unless they depart from the scope of the claims annexed hereto, to be construed as included therein.

## Claims

1. A blood processing system for purifying a plasma in a blood without using a plasma storage bag which comprises:
a plasma separating unit (1) for separating a plasma from a blood;
a plasma purifying unit (7; 8) for purifying the separated plasma;
a plasma introducing fluid circuit (3) for introducing the separated plasma into the plasma purifying unit (7; 8);
a plasma feed pump (5) disposed in the plasma introducing fluid circuit (3) and having a plasma intake port and a plasma discharge port;
a plasma inlet pressure gauge (23) for measuring a pressure of the plasma at the plasma intake port of the plasma feed pump (5);
a plasma return fluid circuit (19) for returning the plasma, which has been purified by the plasma purifying unit (7; 8), back to the plasma separating unit (1);
a bypass fluid circuit (22) extending between the plasma introducing fluid circuit (3) and the plasma return fluid circuit (13) for bypassing the plasma feed pump (5) and the plasma purifying unit (7; 8);
a valve (21) disposed in the bypass circuit (22) for opening the bypass circuit (22) in the event of an occurrence of operative abnormality in the blood purifying system; and
a control unit (26) for controlling the plasma feed pump (5) in reference to the plasma inlet pressure measured by the plasma inlet pressure gauge (23) to render the plasma inlet pressure to fall within a predetermined range with respect to a preset pressure.

2. The blood processing system as claimed in Claim 1, wherein the control unit (26) controls the plasma feed pump (5) to render the plasma inlet pressure to fall within the range of ±5 mmHg with respect to the preset pressure.

3. The system as claimed in Claim 1 or 2, wherein the control unit (26) controls the plasma feed pump (5) to render the plasma discharged from the plasma feed pump (5) to be within a range of ±5 ml/min. with respect to the flow of the plasma from the plasma separating unit.

4. The system as claimed in Claim 1, 2, or 3 comprising a pressure gauge (24) disposed between the plasma feed pump (5) and the plasma purifying unit (7;8) for detecting a plasma purifier pressure and wherein in the event that the plasma purifier pressure exceeds a predetermined value, the control unit (26) halts the plasma feed pump (5) and causes the valve (21) on the bypass fluid circuit (22) to open.

5. The system as claimed in Claim 1, 2,3 or 4 comprising a pressure gauge (25) disposed in the plasma return fluid circuit (19) for detecting a pressure of the plasma being returned and wherein in the event that the pressure of the plasma being returned exceeds a predetermined value the control unit (26) halts the plasma feed pump (5) and causes the valve (21) on the bypass fluid circuit (22) to open.

6. The system as claimed in any one of Claims 1 to 5, wherein in the event that the plasma feed pump (5) is halted, the control unit (26) causes the valve (21) on the bypass fluid circuit (22) to open.

7. The system as claimed in any one of Claims 1 to 6, wherein the plasma purifying unit is selected from the group consisting of a plasma component fractionating membrane module (7) and a plasma component adsorbent unit (8).

8. The blood processing system as claimed in Claim 7, wherein the plasma purifying unit comprises the plasma component fractionating membrane module (7), and further comprising a filtrate feed pump (11) operable in association with the plasma feed pump (5) for draining a filtrate separated from the plasma by the plasma component fractionating membrane module (7).

9. The blood processing system as claimed in Claim 8, further comprising a supplementary liquid supply circuit (14) for supplying a supplementary liquid to the plasma component fractionating membrane module (7) by means of the filtrate feed pump (11) to thereby supplement a filtrated plasma with the supplementary liquid in a quantity corresponding to the quantity of the filtrate separated from the plasma.

## Patentansprüche

1. Blutbehandlungssystem zum Reinigen von Plasma in Blut ohne Gebrauch eines Plasmabeutels, das aufweist:
eine Plasmatrenneinheit (1) zur Plasmatrennung von Blut;
eine Plasmareinigungseinheit (7; 8) zum Reinigen des getrennten Plasmas;
einen Plasmaeinleitungsfluidkreis (3) zum Einleiten des getrennten Plasmas in die Plasmareinigungseinheit (7; 8);
eine Plasmaförderpumpe (5), die im Plasmaeinleitungsfluidkreis (3) angeordnet ist und einen Plasmaeingangsanschluß und einen Plasmaabgabeanschluß hat;
einen Plasmaeinlaßdruckmesser (23) zum Messen eines Drucks des Plasmas am Plasmaeingangsanschluß der Plasmaförderpumpe (5);
einen Plasmarücklauffluidkreis (19) zum Rückführen des Plasmas, das durch die Plasmareinigungseinheit (7; 8) gereinigt wurde, zur Plasmatrenneinheit (1);
einen Umgehungsfluidkreis (22), der sich zwischen dem Plasmaeinleitungsfluidkreis (3) und dem Plasmarücklauffluidkreis (19) erstreckt, zum Umgehen der Plasmaförderpumpe (5) und der Plasmareinigungseinheit (7; 8);
ein Ventil (21), das im Umgehungskreis (22) angeordnet ist, zum Öffnen des Umgehungskreises (22) beim Auftreten einer Betriebsanomalie im Blutreinigungssystem; und
eine Steuereinheit (26) zum Steuern der Plasmaförderpumpe (5) anhand des Plasmaeinlaßdrucks, der durch den Plasmaeinlaßdruckmesser (23) gemessen wird, um den Plasmaeinlaßdruck in einen vorbestimmten Bereich im Hinblick auf einen voreingestellten Druck fallen zu lassen.

2. Blutbehandlungssystem nach Anspruch 1, wobei die Steuereinheit (26) die Plasmaförderpumpe (5) steuert, um den Plasmaeinlaßdruck in den Bereich von ± 5 mmHg im Hinblick auf den voreingestellten Druck fallen zu lassen.

3. System nach Anspruch 1 oder 2, wobei die Steuereinheit (26) die Plasmaförderpumpe (5) steuert, um das von der Plasmaförderpumpe (5) abgegebene Plasma in einem Bereich von ± 5 ml/min im Hinblick auf den Durchfluß des Plasmas von der Plasmatrenneinheit liegen zu lassen.

4. System nach Anspruch 1, 2 oder 3 mit einem zwischen der Plasmaförderpumpe (5) und der Plasmareinigungseinheit (7; 8) angeordneten Druckmesser (24) zum Detektieren eines Plasmareinigerdrucks und wobei bei Übersteigen eines vorbestimmten Werts durch den Plasmareinigerdruck die Steuereinheit (26) die Plasmaförderpumpe (5) stoppt und veranlaßt, daß das Ventil (21) am Umgehungsfluidkreis (22) öffnet.

5. System nach Anspruch 1, 2, 3 oder 4 mit einem im Plasmarücklauffluidkreis (19) angeordneten Druckmesser (25) zum Detektieren eines Drucks des rückgeführten Plasmas und wobei bei Übersteigen eines vorbestimmten Werts durch den Druck des rückgeführten Plasmas die Steuereinheit (26) die Plasmaförderpumpe (5) stoppt und veranlaßt, daß das Ventil (21) am Umgehungsfluidkreis (22) öffnet.

6. System nach einem der Ansprüche 1 bis 5, wobei bei Stopp der Plasmaförderpumpe (5) die Steuereinheit (26) veranlaßt, daß das Ventil (21) am Umgehungsfluidkreis (22) öffnet.

7. System nach einem der Ansprüche 1 bis 6, wobei die Plasmareinigungseinheit aus der Gruppe ausgewählt ist, die aus einem Plasmakomponenten-Fraktioniermembranmodul (7) und einer Plasmakomponenten-Adsorbenseinheit (8) besteht.

8. Blutbehandlungssystem nach Anspruch 7, wobei die Plasmareinigungseinheit das Plasmakomponenten-Fraktioniermembranmodul (7) aufweist und ferner mit einer Filtratförderpumpe (11), die in Zuordnung zur Plasmaförderpumpe (5) zum Ablassen eines Filtrats betreibbar ist, das durch das Plasmakomponenten-Fraktioniermembranmodul (7) vom Plasma getrennt ist.

9. Blutbehandlungssystem nach Anspruch 8, ferner mit einem Zusatzflüssigkeitszufuhrkreis (14) zum Zuführen einer Zusatzflüssigkeit zum Plasmakomponenten-Fraktioniermembranmodul (7) mit Hilfe der Filtratförderpumpe (11), um **dadurch** filtriertes Plasma mit der Zusatzflüssigkeit in einer Menge zu ergänzen, die der Menge des vom Plasma getrennten Filtrats entspricht.

## Revendications

1. Système de traitement du sang pour purifier le plasma dans le sang, sans utiliser de sac de stockage de plasma, qui comprend :
une unité de séparation de plasma (1) pour séparer le plasma du sang ;
une unité de purification de plasma (7 ; 8) pour purifier le plasma séparé ;
un circuit fluide d'introduction de plasma (3) pour introduire le plasma séparé dans l'unité de purification de plasma (7 ; 8) ;
une pompe d'alimentation de plasma (5) disposée dans le circuit fluide d'introduction de plasma (3) et ayant un orifice d'admission de plasma et un orifice d'évacuation de plasma ;
un indicateur de pression d'entrée du plasma (23) pour mesurer la pression du plasma à l'orifice d'entrée de plasma de la pompe d'alimentation de plasma (5) ;
un circuit fluide de retour de plasma (19) pour renvoyer le plasma, qui a été purifié par l'unité de purification de plasma (7 ; 8), à l'unité de séparation de plasma (1) ;
un circuit fluide de dérivation (22), s'étendant entre le circuit fluide d'introduction de plasma (3) et le circuit fluide de retour de plasma (19), pour contourner la pompe d'alimentation de plasma (5) et l'unité de purification de plasma (7 ; 8) ;
une soupape (21) disposée dans le circuit de dérivation (22) pour ouvrir le circuit de dérivation (22) en cas de survenue d'une anomalie opérationnelle dans le système de purification du sang ; et
une unité de commande (26) pour réguler la pompe d'alimentation de plasma (5) en se référant à la pression d'entrée du plasma, mesurée par l'indicateur de pression d'entrée de plasma (23), afin que la pression d'entrée du plasma reste dans une gamme prédéterminée relativement à une pression préétablie.

2. Système de traitement du sang selon la revendication 1, dans lequel l'unité de commande (26) régule la pompe d'alimentation de plasma (5) pour faire en sorte de maintenir la pression d'entrée du plasma dans la gamme de ± 5 mmHg relativement à la pression préétablie.

3. Système selon la revendication 1 ou 2, dans lequel l'unité de commande (26) régule la pompe d'alimentation de plasma (5) de sorte que le plasma évacué de la pompe d'alimentation de plasma (5) soit dans une gamme de ± 5 ml/min, relativement au flux de plasma provenant de l'unité de séparation de plasma.

4. Système selon la revendication 1, 2 ou 3 comprenant un indicateur de pression (24), disposé entre la pompe d'alimentation de plasma (5) et l'unité de purification de plasma (7 ; 8), afin de détecter la pression du purificateur de plasma et dans lequel, dans le cas où la pression du purificateur de plasma dépasserait une valeur prédéterminée, l'unité de commande (26) arrête la pompe d'alimentation de plasma (5) et provoque l'ouverture de la soupape (21) sur le circuit fluide de dérivation (22).

5. Système selon la revendication 1, 2, 3, ou 4, comprenant un indicateur de pression (25) disposé dans le circuit fluide de retour de plasma (19), afin de détecter la pression du plasma renvoyé et dans lequel, dans le cas où la pression du plasma renvoyé dépasserait une valeur prédéterminée, l'unité de commande (26) arrête la pompe d'alimentation de plasma (5) et provoque l'ouverture de la soupape (21) sur le circuit fluide de dérivation (22).

6. Système selon l'une quelconque des revendications 1 à 5, dans lequel, dans le cas où la pompe d'alimentation de plasma (5) serait arrêtée, l'unité de commande (26) provoque l'ouverture de la soupape (21) sur le circuit fluide de dérivation (22).

7. Système selon l'une quelconque des revendications 1 à 6, dans lequel l'unité de purification de plasma est choisie dans le groupe constitué d'un module à membrane de fractionnement de composant de plasma (7) et d'une unité adsorbante de composant de plasma (8).

8. Système de traitement du sang selon la revendication 7, dans lequel l'unité de purification de plasma comprend le module à membrane de fractionnement du composant de plasma (7), et comprend en outre une pompe d'alimentation de filtrat (11) pouvant être actionnée en association avec la pompe d'alimentation de plasma (5), afin de drainer un filtrat séparé du plasma, par le biais du module à membrane de fractionnement du composant de plasma (7).

9. Système de traitement du sang selon la revendication 8, comprenant en outre un circuit d'alimentation de liquide supplémentaire (14), pour fournir un liquide supplémentaire à un module à membrane de fractionnement du composant de plasma (7), au moyen de la pompe d'alimentation de filtrat (11), afin de compléter ainsi un plasma filtré avec le liquide supplémentaire dans une quantité correspondant à la quantité du filtrat séparé du plasma.
